(19) Europäisches Patentamt / European Patent Office / Office européen des brevets

(11) **EP 2 700 448 A1**

(12) **EUROPÄISCHE PATENTANMELDUNG**

(43) Veröffentlichungstag:
**26.02.2014 Patentblatt 2014/09**

(21) Anmeldenummer: **12181153.3**

(22) Anmeldetag: **21.08.2012**

(51) Int Cl.:
*B01J 39/04* (2006.01)    *B01J 39/16* (2006.01)
*B01D 11/04* (2006.01)    *C02F 1/42* (2006.01)
*C07C 227/40* (2006.01)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(71) Anmelder: **Evonik Industries AG**
**45128 Essen (DE)**

(72) Erfinder:
• **Erhardt, Frank**
  **33604 Bielefeld (DE)**
• **Falke, Lukas**
  **33602 Bielefeld (DE)**
• **Kelle, Ralf**
  **33330 Gütersloh (DE)**
• **Häger, Harald**
  **59348 Lüdinghausen (DE)**
• **Haas, Thomas**
  **48161 Münster (DE)**
• **Hennemann, Hans-Georg**
  **45770 Marl (DE)**
• **Thum, Oliver**
  **40880 Ratingen (DE)**
• **Roos, Martin**
  **45721 Haltern am See (DE)**
• **Pötter, Markus**
  **48149 Münster (DE)**

(54) **Verzweigte Fettsäuren als flüssige Kationenaustauscher**

(57)    Die vorliegende Erfindung betrifft ein Verfahren zum Entfernen einer organischen Verbindung aus einer wässrigen Lösung, umfassend die Schritte Bereitstellen der wässrigen Lösung, die die organische Verbindung enthält, und einer hydrophoben organischen Lösung, wobei letztere einen flüssigen hydrophoben Kationenaustauscher umfasst, Kontaktieren der wässrigen Lösung und der hydrophoben organischen Lösung, und Abtrennen der hydrophoben organischen Lösung von der wässrigen Lösung, wobei es sich bei dem flüssigen hydrophoben Kationenaustauscher um eine gesättigte Alkansäure mit wenigstens einem Alkylsubstituenten handelt, wobei es sich bei der organischen Verbindung um eine organische Verbindung mit wenigstens einer positiven Ladung und einer neutralen oder positiven Gesamtladung handelt.

EP 2 700 448 A1

**Beschreibung**

Die vorliegende Erfindung betrifft ein Verfahren zum Entfernen einer organischen Verbindung aus einer wässrigen Lösung, umfassend die Schritte Bereitstellen der wässrigen Lösung, die die organische Verbindung enthält, und einer hydrophoben organischen Lösung, wobei letztere einen flüssigen hydrophoben Kationenaustauscher umfasst, Kontaktieren der wässrigen Lösung und der hydrophoben organischen Lösung, und Abtrennen der hydrophoben organischen Lösung von der wässrigen Lösung, wobei es sich bei dem flüssigen hydrophoben Kationenaustauscher um eine gesättigte Alkansäure mit wenigstens einem Alkylsubstituenten handelt, wobei es sich bei der organischen Verbindung um eine organische Verbindung mit wenigstens einer positiven Ladung und einer neutralen oder positiven Gesamtladung handelt.

Ein grundlegendes Problem bei der von nachwachsenden Rohstoffen ausgehenden biotechnologischen Herstellung von Feinchemikalien, die sonst ausgehend von fossilen Brennstoffen synthetisiert werden, besteht darin, das einmal erhaltene Produkt, das typischerweise in einer großvolumigen wässrigen Phase vorliegt, in eine organische Phase zu überführen. Diese Überführung führt man einerseits durch, um ein fertiges Zwischen- oder Endprodukt zu konzentrieren und um ggf. die synthetische Verarbeitung in folgenden Reaktionsschritten in organischer Lösung zu ermöglichen, andererseits, um die Ausbeute der Reaktion in der wässrigen Phase durch das Entfernen des erwünschten Produktes zu verbessern oder den Ablauf der Reaktion in einem technisch sinnvollen Rahmen überhaupt erst zu ermöglichen. Die direkte thermische Aufkonzentrierung des häufig in niedrigen Konzentrationen vorliegenden Produktes aus der großvolumigen wässrigen Lösung ist in der Regel nicht sinnvoll.

Die Verteilung einer Verbindung in einem Zweiphasensystem im Gleichgewicht umfassend eine wässrige, hydrophile Phase und eine organische, hydrophobe Phase, die sich nicht mischen, hängt maßgeblich von den physikochemischen Eigenschaften der jeweiligen Verbindung ab. Während Verbindungen mit einem hohen Anteil an oder ausschließlich bestehend aus unsubstituierten Kohlenwasserstoffen sich überwiegend in der hydrophoben Phase anreichern, liegen Verbindung mit einem hohen Anteil an polaren Gruppen wie heteroatomhaltigen Funktionalitäten und ganz besonders Verbindungen mit Ladungen überwiegend oder praktisch ausschließlich in der wässrigen Phase vor, was eine Überführung in eine organische Phase erschwert.

Die Verteilung einer Verbindung in dem genannten Zweiphasensystem nach Einstellung des Gleichgewichts wird häufig mit Hilfe von Verteilungskoeffizienten, beispielsweise nach der Nernst'schen Gleichung

$$\alpha = c_{\text{Phase 1}} / c_{\text{Phase 2,}}$$

beschrieben. Ein spezieller Verteilungskoeffizient ist $K_{ow}$, auch als P-Wert bezeichnet, der das Verteilungsgleichgewicht einer Verbindung zwischen einer Oktanol- und einer wässrigen Phase charakterisiert:

$$K_{ow} = P = c_{\text{Oktanol}} / C_{\text{Wasser}}$$

Beispiele für industriell stark nachgefragte, in wässrigem Lösung bei physiologischem pH positiv geladene organischen Verbindung stellen 12-Aminolaurinsäure (ALS) und deren Derivate, insbesondere der Methylester (ALSME), dar. ALS ist ein wichtiges Ausgangsprodukt bei der Herstellung von PolymerenHerkömmlich wird ALS ausgehend von fossilen Rohstoffen in einem Prozess mit niedriger Ausbeute über Laurinlactam hergestellt, das durch Trimerisierung von Butadien, anschließende Hydrierung unter Bildung von Cyclododecan, anschließende Oxidation zu Cyclododecanon, Umsetzung mit Hydroxylaurin und anschließender Beckmann-Umlagerung synthetisiert wird. Ein vielversprechender Weg zur biotechnologischen Herstellung von ALS bzw. ALSME ist in der DE10200710060705 beschrieben.

Der Stand der Technik lehrt die Gewinnung positiv geladener organischer Verbindungen durch Kontaktieren einer wässrigen Reaktionsmischung umfassend eine stoffwechselaktive Zelle mit einer organischen Phase umfassend ein organisches Lösungsmittel. So beschreibt beispielsweise DE10200710060705 die Gewinnung des Produktes ALSME durch Ausschütteln mit Essigsäureethylester aus einem wässrigen Reaktionsgemisch. Asano *et al.* (2008) offenbaren die Extraktion von ALS mit Toluol aus einer wässrigen Reaktionslösung umfassend ein ALS synthetisierendes Enzym (Asano, Y., Fukuta, y., Yoshida, Y., and Komeda, H. (2008): The Screening, Characterisation, and Use of ω-Laurolactam Hydrolase: A New Enzymatic Synthesis of 12-Aminolauric Acid, Biosc. Biotechn. Biochem., 72 (8), 2141-2150).

Der vorliegenden Erfindung liegt daher die Aufgabe zugrunde, ein Verfahren zur Entfernung positiv geladener organischer Verbindungen, besonders ω-Aminocarbonsäuren, mit wenigstens einer positiven Ladung aus einer wässrigen Reaktionsmischung zu entwickeln, wobei eine möglichst vorteilhafte Lage der Verteilungsgleichgewichtes zwischen Reaktionsmischung und einer als Extraktionsmittel verwendeten hydrophoben organischen Phase erwünscht ist, d. h. das Verteilungsgleichgewicht soll möglichst weit auf der Seite der hydrophoben organischen Phase liegen.

Eine weitere der Erfindung zugrunde liegende Aufgabe besteht darin, ein Verfahren zur Entfernung organischer Verbindungen mit wenigstens einer positiven Ladung, besonders ω-Aminocarbonsäuren, aus einer wässrigen Lösung umfassend eine stoffwechselaktive Zelle unter Verwendung einer hydrophoben organischen Phase als Extraktionsmittel zu entwickeln, bei dem das Verteilungsgleichgewicht möglichst weit auf der Seite der hydrophoben organischen Phase liegt.

Eine weitere der Erfindung zugrunde liegende Aufgabe besteht darin, ein Verfahren zur Entfernung organischer Verbindungen mit wenigstens einer positiven Ladung, besonders ω-Aminocarbonsäuren, aus einer wässrigen Lösung unter Verwendung einer hydrophoben organischen Lösung als Extraktionsmittel zu entwickeln, das das Wachstum von biotechnologisch relevanten Mikroorganismen, insbesondere *Escherichia coli,* möglichst wenig beeinträchtigt bzw. verlangsamt und/oder die Zahl teilungsfähiger und/oder lebensfähiger und/oder respiratorisch aktiver und/oder stoffwechsel- und synthetisch aktiver Zellen dabei möglichst wenig verringert.

Eine weitere der Erfindung zugrunde liegende Aufgabe besteht darin, ein Verfahren zur Entfernung organischer Verbindungen mit wenigstens einer positiven Ladung, besonders ω-Aminocarbonsäuren, aus einer wässrigen Lösung unter Verwendung einer hydrophoben organischen Lösung als Extraktionsmittel zu entwickeln, bei der die Haltbarkeit und/oder die Wiedergewinnbarkeit, besonders mit Hinblick auf gleichbleibenden Gehalt flüssigem hydrophoben Kationenaustauscher oder Reinheit des Kationenaustauschers nach Wiedergewinnung, der hydrophoben organischen Lösung möglichst gut ist.

Schließlich liegt der Erfindung die Aufgabe zugrunde, ein Verfahren zur Entfernung einer organischen Verbindung mit wenigstens einer positiven Ladung, besonders ω-Aminocarbonsäuren, aus einer wässrigen Lösung umfassend eine stoffwechselaktive Zelle unter Verwendung einer hydrophoben organischen Phase als Extraktionsmittel aufzufinden, bei dem die Gesamtheit der für die Ausbeute, den Gesamtumsatz und schnelle Durchführbarkeit eines zugrundeliegenden biotechnologischen Syntheseverfahrens entscheidenden Eigenschaften, insbesondere die Toxizität der organischen Phase gegenüber einer stoffwechselaktive Zelle und die Aufnahme der Verbindung in das organische Extraktionsmittel, mit Hinblick auf die Gesamtausbeute oder einen schnelleren Ablauf oder, im Falle eines kontinuierlichen Prozesses, eine möglichst lange Verwendbarkeit der stoffwechselaktiven Zelle optimiert ist, besonders für den Fall, dass die organische Verbindung mit wenigstens einer positiven Ladung das Produkt oder ein Zwischenprodukt des Syntheseverfahrens darstellt, das unter Beteiligung einer katalytischen Aktivität der stoffwechselaktiven Zelle synthetisiert wird.

Diese und weitere Aufgaben werden durch den Gegenstand der vorliegenden Anmeldung und insbesondere auch durch den Gegenstand der beigefügten unabhängigen Ansprüche gelöst, wobei sich Ausführungsformen aus den Unteransprüchen ergeben.

In einem ersten Aspekt wird das der Erfindung zugrunde liegende Problem gelöste durch ein Verfahren zum Entfernen einer organischen Verbindung aus einer wässrigen Lösung, umfassend die Schritte

a) Bereitstellen der wässrigen Lösung, die die organische Verbindung enthält, und einer hydrophoben organischen Lösung, wobei letztere einen flüssigen hydrophoben Kationenaustauscher umfasst,

b) Kontaktieren der wässrigen Lösung und der hydrophoben organischen Lösung, und

c) Abtrennen der hydrophoben organischen Lösung von der wässrigen Lösung,

wobei es sich bei dem flüssigen hydrophoben Kationenaustauscher um eine gesättigte Alkansäure mit wenigstens einem Alkylsubstituenten handelt, wobei es sich bei der organischen Verbindung um eine organische Verbindung mit wenigstens einer positiven Ladung und einer neutralen oder positiven Gesamtladung handelt.

In einer ersten Ausführungsform des ersten Aspekts wird das Problem gelöst durch ein Verfahren, wobei es sich bei der organischen Verbindung um eine Verbindung der Formel

$$NR^2R^3R^4H^+\text{-}A\text{-}COOR^1 \qquad (I)$$

oder

$$NR^2R^3R^4H^+\text{-}A\text{-} NR^5R^6R^7H^+ \qquad (II),$$

handelt,
wobei $R^1$ Wasserstoff, Methyl, Ethyl, Propyl, Isopropyl oder eine negative Ladung ist,
wobei A eine Alkylengruppe mit wenigstens drei, bevorzugt wenigstens sechs, besonders bevorzugt acht Kohlenstoffatomen ist, die bevorzugt unsubstituiert und geradkettig ist,

und wobei $R^2$, $R^3$, $R^4$, $R^5$, $R^6$ und $R^7$ jeweils und unabhängig voneinander aus der Gruppe ausgewählt sind, die Wasserstoff, Methyl, Ethyl und Propyl umfasst.

In einer zweiten Ausführungsform des ersten Aspekts, bei der es sich auch um eine Ausführungsform der ersten Ausführungsform handelt, wird das Problem gelöst durch ein Verfahren, wobei der pH-Wert in der wässrigen Lösung bei Schritt b) 6 bis 8, bevorzugt 6,2 bis 7,2, beträgt.

In einer dritten Ausführungsform des ersten Aspekts, bei der es sich auch um eine Ausführungsform der ersten bis zweiten Ausführungsform handelt, wird das Problem gelöst durch ein Verfahren, wobei das Stoffmengenverhältnis von flüssigem Kationenaustauscher zu organischer Verbindung bei Schritt b) wenigstens 1 beträgt.

In einer vierten Ausführungsform des ersten Aspekts, bei der es sich auch um eine Ausführungsform der ersten bis dritten Ausführungsform handelt, wird das Problem gelöst durch ein Verfahren, wobei das Volumenverhältnis von organischer Lösung zu wässriger Lösung 1:10 bis 10:1 beträgt.

In einer fünften Ausführungsform des ersten Aspekts, bei der es sich auch um eine Ausführungsform der ersten bis vierten Ausführungsform handelt, wird das Problem gelöst durch ein Verfahren, wobei der flüssige Kationenaustauscher eine verzweigte Fettsäure der Formel $(H_3C)_2CH-(CH_2)_n-COOH$ oder eine unprotonierte Form davon ist und n wenigstens 4, bevorzugt wenigstens 8 beträgt und am bevorzugtesten 14 ist.

In einer sechsten Ausführungsform des ersten Aspekts, bei der es sich auch um eine Ausführungsform der ersten bis fünften Ausführungsform handelt, wird das Problem gelöst durch ein Verfahren, wobei der flüssige Kationenaustauscher eine gesättigte Alkansäure mit wenigstens einem Alkylsubstituent ist, die bevorzugt insgesamt wenigstens 12 Kohlenstoffatome umfasst.

In einer siebten Ausführungsform des ersten Aspekts, bei der es sich auch um eine Ausführungsform der ersten bis sechsten Ausführungsform handelt, wird das Problem gelöst durch ein Verfahren wobei die wässrige Lösung weiterhin eine stoffwechselaktive Zelle umfasst.

In einer achten Ausführungsform des ersten Aspekts, bei der es sich auch um eine Ausführungsform der ersten bis siebten Ausführungsform handelt, wird das Problem gelöst durch ein Verfahren, wobei es sich bei der organischen Verbindung um eine für eine Zelle, bevorzugt eine bakterielle Zelle, toxische Verbindung handelt.

In einer neunten Ausführungsform des ersten Aspekts, bei der es sich auch um eine Ausführungsform der ersten bis achten Ausführungsform handelt, wird das Problem gelöst durch ein Verfahren, wobei die organische Lösung darüber hinaus mindestens ein organisches Lösungsmittel enthält, bevorzugt eine Fettsäure und/oder einen Fettsäureester.

In einer zehnten Ausführungsform des ersten Aspekts, bei der es sich auch um eine Ausführungsform der ersten bis neunten Ausführungsform handelt, wird das Problem gelöst durch ein Verfahren, wobei die hydrophobe organische Lösung den flüssigen hydrophoben Kationenaustauscher in einem Volumenanteil von 20 bis 80 %, bevorzugt 25 bis 75 % enthält. In einer elften Ausführungsform des zweiten Aspekts, bei der es sich auch um eine Ausführungsform der ersten bis zehnten Ausführungsform des ersten Aspekts handelt, ist die organische Verbindung ein Diamin ausgewählt aus der Gruppe, die Butandiamin, 1,5-Pentandiamin, 1,6-Hexandiamin, 1,8-Octandiamin, 1,14-Tetradecandiamin, 1,18-Octadecandiamin, 2-Methyl-1,5-Diaminopentan, 2,2-Dimethyl-1,5-diaminopentan, 4,4'-Diaminodicyclohexylmethan, 3,3'-Dimethyl-4,4'-diaminodicyclohexylmethan, 3,3',5,5'-Tetramethyl-4,4'-diaminodicyclohexylmethan, m- oder p-Xylylendiamin, 2.2.4- oder 2.4.4-Trimethylhexamethylendiamin, 1.4-Diaminocyclohexan, 4.4'-Diaminodicyclohexylpropan, Isophorondiamin, Metaxylylendiamin und Paraxylylendiamin umfasst.

In einer zwölften Ausführungsform des zweiten Aspekts, bei der es sich auch um eine Ausführungsform der ersten bis elften Ausführungsform handelt, liegt die Temperatur bei Schritt b) zwischen 28 und 70 °C, bevorzugt zwischen 30 und 37 °C.

In einem zweiten Aspekt wird das der Erfindung zugrunde liegende Problem gelöst durch eine Reaktionsmischung umfassend eine wässrige Lösung, die eine organische Verbindung enthält, und eine hydrophobe organische Lösung,

wobei die hydrophobe organische Lösung einen flüssigen hydrophoben Kationenaustauscher umfasst,

wobei es sich bei dem flüssigen hydrophoben Kationenaustauscher um eine gesättigte Alkansäure mit wenigstens einem Alkylsubstituenten handelt,

und wobei es sich bei der organischen Verbindung um eine organische Verbindung mit wenigstens einer positiven Ladung und einer neutralen oder positiven Gesamtladung handelt.

In einer ersten Ausführungsform des zweiten Aspekts wird das Problem gelöst durch eine Reaktionsmischung, wobei es sich bei der organischen Verbindung um eine Verbindung der Formel

$$NR^2R^3R^4H^+\text{-A-COOR}^1 \qquad \text{(I)}$$

oder

$$NR^2R^3R^4H^+\text{-A- }NR^5R^6R^7H^+ \qquad \text{(II)},$$

wobei $R^1$ Wasserstoff, Methyl, Ethyl, Propyl, Isopropyl oder eine negative Ladung ist,

wobei A eine Alkylengruppe mit wenigstens drei, bevorzugt wenigstens sechs, besonders bevorzugt acht Kohlenstoffatomen ist, die bevorzugt unsubstituiert und geradkettig ist,

und wobei $R^2$, $R^3$, $R^4$, $R^5$, $R^6$ und $R^7$ jeweils und unabhängig voneinander aus der Gruppe ausgewählt sind, die Wasserstoff, Methyl, Ethyl und Propyl umfasst.

In einer zweiten Ausführungsform des zweiten Aspekts, bei der es sich auch um eine Ausführungsform der ersten Ausführungsform des ersten Aspekts handelt, wird das Problem gelöst durch eine Reaktionsmischung, wobei der flüssige hydrophobe Kationenaustauscher eine verzweigte Fettsäure der Formel $(H_3C)_2CH-(CH_2)_n-COOH$ oder eine unprotonierte Form davon ist und n wenigstens 4, bevorzugt wenigstens 8 beträgt und am bevorzugtesten 14 ist.

In einer dritten Ausführungsform des zweiten Aspekts, bei der es sich auch um eine Ausführungsform der ersten bis zweiten Ausführungsform des ersten Aspekts handelt, wobei der flüssige Kationenaustauscher eine gesättigte Alkansäure mit wenigstens einem Alkylsubstituenten ist, die bevorzugt insgesamt wenigstens 12 Kohlenstoffatome umfasst.

In einer vierten Ausführungsform des zweiten Aspekts, bei der es sich auch um eine Ausführungsform der ersten bis dritten Ausführungsform des ersten Aspekts handelt, wobei die wässrige Lösung weiterhin eine stoffwechselaktive Zelle umfasst.

In einer fünften Ausführungsform des zweiten Aspekts, bei der es sich auch um eine Ausführungsform der ersten bis vierten Ausführungsform des zweiten Aspekts handelt, ist die organische Verbindung ein Diamin ausgewählt aus der Gruppe, die Butandiamin, 1,5-Pentandiamin, 1,6-Hexandiamin, 1,8-Octandiamin, 1,14-Tetradecandiamin, 1,18-Octadecandiamin, 2-Methyl-1,5-Diaminopentan, 2,2-Dimethyl-1,5-diaminopentan, 4,4'-Diaminodicyclohexylmethan, 3,3'-Dimethyl-4,4'-diaminodicyclohexylmethan, 3,3',5,5'-Tetramethyl-4,4'-diaminodicyclohexylmethan, m- oder p-Xylylendiamin, 2.2.4- oder 2.4.4-Trimethylhexamethylendiamin, 1.4-Diaminocyclohexan, 4.4'-Diaminodicyclohexylpropan, Isophorondiamin, Metaxylylendiamin und Paraxylylendiamin umfasst.

Weitere Ausführungsformen des zweiten Aspekts umfassen sämtliche Ausführungsformen des ersten Aspekts der vorliegenden Erfindung.

Die Erfinder der vorliegenden Erfindung haben herausgefunden, dass die Effizienz der Entfernung einer organischen Verbindung mit einer positiven Ladung aus einer wässrigen Lösung in eine hydrophobe organische Lösung überraschend gesteigert werden kann, wenn diese organische Lösung einen flüssigen Kationenaustauscher umfasst, bei dem es sich um eine gesättigten Alkansäure mit wenigstens einem Alkylsubstituenten handelt. Ohne an irgendeine Theorie gebunden sein zu wollen, vermuten die Erfinder der vorliegenden Erfindung, dass die negative Ladung bzw. die negativen Ladungen des flüssigen Kationenaustauscher mit der einen positiven Ladung oder den mehreren positiven Ladungen der organischen Verbindungen ionisch interagiert/interagieren und dass diese Interaktion zu einer Maskierung wenigstens einer positiven Ladung führt, welche die Löslichkeit in der organischen Phase erhöht.

Die vorliegende Erfindung sieht die Verwendung einer gesättigten Alkansäure mit wenigstens einem Alkylsubstituenten als flüssiger hydrophober Kationenaustauscher zur Entfernung, einer organischen Verbindung mit einer positiven Ladung aus einer wässrigen Lösung vor, bevorzugt unter Akkumulierung der organischen Verbindung in der hydrophoben organischen Lösung. In einer bevorzugten Ausführungsform wird unter dem Begriff "gesättigten Alkansäure mit wenigstens einem Alkylsubstituenten" eine Alkansäure der Formel $CH_3-(CH_2)_n-COOH$ oder eine unprotonierte Form davon verstanden, bei der wenigstens ein Wasserstoffatom aus der Alkylkette gegen einen Alkylsubstituenten der Formel $-(CH_2)_m-H$ ausgetauscht ist, wobei n und m jeweils und unabhängig voneinander 1, 2, 3, 4, 5, 6, 7, 8, 9, 10, 11, 12, 13, 14, 15, 16, 17, 18, 19, 20, 21, 22, 23, 24, 25, 26, 27, 28, 29 oder 30 sein können. In einer bevorzugtesten Ausführungsform handelt es sich bei der gesättigten Alkansäure mit wenigstens einem Alkylsubstituenten um eine Verbindung aus der Gruppe umfassend Isostearinsäure, Isopalmitinsäure, Isomyristinsäure, Phytansäure, 2-Hexyldekansäure, 2-Butyloktansäure, 15-Methylhexadekansäure, 13-Methyltetradekansäure handelt. In einer besonders bevorzugten Ausführungsform ist das vorletzte Kohlenstoffatom in der Alkylkette substituiert, besonders bevorzugt mit einer Methylgruppe. Erfindungsgemäß verwendbar sind ausdrücklich Mischungen verschiedener gesättigter Alkansäuren mit wenigstens einem Alkylsubstituenten, optional mit weiteren, strukturell von letzteren verschiedenen flüssigen hydrophoben Kationenaustauschern.

Wie sämtliche Verbindungen in dieser Anmeldung umfasst eine gesättigte Alkansäure mit wenigstens einem Alkylsubstituenten in einer bevorzugten Ausführungsform gleichermaßen unprotonierte, teilweise und vollständig protonierte Formen der Verbindung.

Die erfindungsgemäße Lehre ist zur Entfernung strukturell diverser organischer Verbindungen mit einer positiven Ladung und positiver oder neutraler Gesamtladung aus einer wässrigen Lösung geeignet. In einer bevorzugten Ausführungsform handelt es sich bei der organischen Verbindung um eine Verbindung der Formel

$$NR^2R^3R^4H^+\text{-A-COOR}^1 \qquad (I)$$

oder

$$NR^2R^3R^4H^+\text{-}A\text{-}\ NR^5R^6R^7H^+ \qquad (II),$$

wobei $R^1$ Wasserstoff, Methyl, Ethyl, Propyl, Isopropyl oder eine negative Ladung ist,
wobei A eine Alkylengruppe mit wenigstens drei, bevorzugt wenigstens sechs, besonders bevorzugt acht Kohlenstoffatomen ist, die bevorzugt unsubstituiert und geradkettig ist. Bei A kann es sich um ein verzweigtes oder unverzweigtes, lineares oder zyklisches Alkan oder einen Aromaten oder Heteroaromaten handeln, das oder der wenigstens mit den beiden in Formel (II) angegebenen Substituenten substituiert ist. In einer besonders bevorzugten Ausführungsform handelt es sich bei der organischen Verbindung um eine Verbindung der Formel II, und A stellt eine Alkylenkette der Formel $-(CH_2)n-$ dar, wobei n 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 oder 40 ist, und besonders bevorzugt ist zusätzlich $R^2=R^3=R^4=R^5=R^6=R^7 = H$. und wobei $R^2$, $R^3$, $R^4$, $R^5$, $R^6$ und $R^7$ jeweils und unabhängig voneinander aus der Gruppe ausgewählt sind, die Wasserstoff, Methyl, Ethyl und Propyl umfasst. In einer bevorzugten Ausführungsform handelt es sich um Aminolaurinsäure oder einen Ester davon, bevorzugt den Methylester.

In einer bevorzugten Ausführungsform bedeutet der Begriff "flüssiger Kationenaustauscher", wie hierin verwendet, eine in einem bei Raumtemperatur flüssigen hydrophoben organischen Lösungsmittel lösliche Verbindung, die aufgrund einer wenigstens teilweise an der Carboxylatgruppe der Alkansäure vorliegenden negativen Ladung in der Lage ist, wenigstens transient eine ionische Wechselwirkung mit wenigstens einem Kation auszubilden.

Bei der wässrigen Lösung, aus der die organische Verbindung entfernt wird, handelt es sich bevorzugt um eine Wasser enthaltene Pufferlösung oder ein wässriges Kulturmedium, die oder das Wasser noch bevorzugter als vorherrschendes Lösungsmittel enthält. Beispielsweise beträgt der Lösungsmittelanteil des Wassers in der wässrigen Lösung mehr als 10, 20, 30, 40, 50, 60, 70, 80, 90, 95, 99 Volumenprozent oder 100 Volumenprozent. Dem Fachmann sind zahlreiche wässrige Puffer Kulturmedien bekannt, die für die Erhaltung oder Kultivierung von Zellen, insbesondere biotechnologisch bedeutsamer Zellen, geeignet sind. Darunter fallen gleichermaßen Vollmedien wie LB-Medien, Minimalmedien wie M9-Medien, Minimalmedien mit Komplexbestandteilen wie Hefeextrakt oder Pepton, Kombinationen aus den zuvor genannten sowie Selektivmedien, beispielsweise solche, die eine hohe Salzkonzentration enthalten und daher nur das Wachstum halophiler oder zumindest halotoleranter Organismen ermöglichen. In einer bevorzugten Ausführungsform wird unter dem Begriff "wässriges Kulturmedium", wie hierin verwendet, ein Reaktionsmedium auf Wasserbasis verstanden, das mit Hinblick auf sämtliche relevante Faktoren, insbesondere pH-Wert, Salzgehalt und Temperatur, derart beschaffen ist, das es die Lebensfähigkeit darin enthaltener Zellen, bevorzugt Mikroorganismen, erhält oder fördert und sowohl wässriges Kulturmedium als auch hydrophobe organische Phase in flüssiger Form vorliegen. Die Temperaturansprüche verschiedener biotechnologisch bedeutsamer Zellen können mikrobiologischen und molekularbiologischen Lehrbüchern entnommen werden, z.B. Fuchs/Schlegel (2007) Allgemeine Mikrobiologie, 2008, Georg Thieme Verlag. In einer bevorzugten Ausführungsform liegt der pH-Wert des wässrigen Kulturmediums zum Zeitpunkt des Kontaktierens zwischen 4 bis 9, bevorzugter zwischen 4,5 bis 8,5, am bevorzugtesten zwischen 6,2 und 7,2. In einer bevorzugten Ausführungsform wird der pH-Wert des wässrigen Kulturmediums bei Schritt b) wenigstens 0,5 h, bevorzugter wenigstens 2 h, noch bevorzugter wenigstens 6 h, am bevorzugtesten wenigstens 12 h im Bereich von pH 4 bis 9, bevorzugter zwischen 4,5 bis 8,5, am bevorzugtesten zwischen 6,2 und 7,2 gehalten. Dem Fachmann ist aus dem Stand bekannt, wie der pH-Wert einer wässrigen Lösung, insbesondere auch einer wässrigen Lösung umfassend stoffwechseaktive Zellen und zu deren Kultur erforderliche Medien, durch Zugabe von Säure oder Base, beispielsweise Schwefelsäure bzw. Ammoniakwasser, eingestellt und geregelt werden kann. In einer weiteren bevorzugten Ausführungsform liegt die Temperatur zwischen 0 und 45 °C, bevorzugter zwischen 15 und 40 °C, am bevorzugtesten zwischen 20 und 37 °C.

In einer bevorzugten Ausführungsform der vorliegenden Erfindung wird unter dem Begriff "Kontaktieren", wie hierin verwendet, verstanden, dass zwei Phasen direkt und insbesondere ohne Zwischenschaltung einer physikalischen Barriere wie einer Membran einander gegenüber exponiert werden. Das Kontaktieren erfolgt im einfachsten Fall dadurch, dass die beiden Phasen ins gleiche Gefäß gegeben und in geeigneter Weise, beispielsweise durch Rühren, mit einander vermischt werden. Ganz ausdrücklich ist für die Ausführung der erfindungsgemäßen Lehre möglich, aber keinesfalls erforderlich, dass ein biphasisches Systems vorliegt. Vielmehr ist eine gute, möglichst kontinuierliche Durchmischung der Summe der wässrigen Lösung und der hydrophoben organischen Lösung, beispielsweise durch Rühren, der Entfernung der organischen Lösung aus der wässrigen Lösung förderlich.

In einer bevorzugten Ausführungsform bedeutet der Begriff "weist eine Ladung auf", wie hierin verwendet, dass eine so charakterisierte Verbindung eine entsprechende Ladung an einer geeigneten funktionellen Gruppe in wässriger Lösung bei pH 0 bis 14, bevorzugte 2 bis 12, 2 bis 6, 8 bis 12, 3 bis 10, 6 bis 8, am bevorzugtesten bei pH 7, aufweist, beispielsweise eine positive Ladung an einer Ammoniumgruppe. In einer bevorzugten Ausführungsform handelt es sich um eine permanent vorliegende Ladung. In einer weiteren bevorzugten Ausführungsform bedeutet der Begriff "weist eine Ladung auf", wie hierin verwendet, dass die entsprechende funktionelle Gruppe oder Verbindung bei pH 7 überwiegend mit der entsprechenden Ladung vorliegt, d.h. zu wenigstens 50, bevorzugter 90 noch bevorzugter 99 %. Beispielsweise weist das Molekül Ethanolamim bei pH 0 eine Ladung auf. Dabei handelt es sich um die protonierte Ammoniumgruppe. In einer bevorzugten Ausführungsform bedeutet der Begriff "Gesamtladung" einer Verbindung hingegen die Summe aller Ladungen an der Verbindung bei pH 0 bis 14, bevorzugte 2 bis 12, 2 bis 6, 8 bis 12, 3 bis 10, 6 bis 8, am bevorzugtesten bei pH 7. Beispielsweise weist die Verbindung Glycin in wässriger Lösung bei pH 6 zwei Ladungen auf, nämlich eine negative an der Carboxyfunktion und eine positive an der protonierten Amingruppe, d. h. insgesamt eine neutrale Gesamtladung.

In einer bevorzugten Ausführungsform der Erfindung ist der Begriff "enthaltend" im Sinne von "umfassend", d.h. nicht abschließend zu verstehen. Eine Mischung enthaltend A kann in diesem Sinne neben A weitere Bestandteile aufweisen. Die Formulierung "eine oder mehrere Ladungen" bedeutet wenigstens eine Ladung der entsprechenden Natur.

In einer bevorzugten Ausführungsform wird unter dem Begriff "hydrophob", wie hierin verwendet, die Fähigkeit einer Flüssigkeit verstanden, in Anwesenheit von einer wässrigen Phase und im Gleichgewicht, d. h. insbesondere in Abwesenheit von gegenwirkenden Maßnahmen wie Rühren, eine eigene, von der wässrigen Phase klar abgegrenzte flüssige Phase auszubilden. Bei letzterer kann es sich um eine zusammenhängende flüssige Phase oder um eine Emulsion handeln. In einer weiteren bevorzugten Ausführungsform wird mit dem Begriff "hydrophob", wie hierin verwendet, die Eigenschaft einer Verbindung verstanden, sich im Wesentlichen nicht in Wasser zu lösen. Schließlich wird der Begriff in einer weiteren bevorzugten Ausführungsform, wie hierin verwendet, derart verstanden, dass eine derartige bezeichnete Verbindung einen P-Wert (J. Sangster, Octanol-Water Partition Coefficients: Fundamentals and Physical Chemistry, Vol. 2 of Wiley Series in Solution Chemistry, John Wiley & Sons, Chichester, 1997) aufweist, dessen dekadischer Logarithmus größer 0, bevorzugter größer 0,5, noch bevorzugter größer 1 und am bevorzugtesten größer 2 ist. Bevorzugte organische Lösungsmittel umfassen, sind aber nicht beschränkt auf Lösungsmittel aus der Gruppe umfassend bei Raumtemperatur flüssige substituierte und nichtsubstituierte Alkane, Cycloalkane, Cycloalkene, Aryle, Fettsäuren, Fettsäureester, Alkohole, Heterocycloalkane, Heterocycloalkene und Heteroaryle umfasst. Die hydrophobe organische Lösung kann auch eine Mischung umfassend mehr als ein hydrophobes organisches Lösungsmittel sein.

In einer weiteren Ausführungsform der vorliegenden Erfindung ist der flüssige lonenaustauscher nicht oder nur eine mäßig toxisch mit Hinblick auf stoffwechselaktive Zellen, besonders biotechnologisch relevante Mikroorganismen. Unter dem Begriff "toxisch", wie hierin verwendet, wird in einer bevorzugten Ausführungsform der Erfindung die Eigenschaft einer Verbindung verstanden, bei Kontakt mit den entsprechenden Mikroorganismen deren Wachstumsgeschwindigkeit zu senken, deren Stoffwechselaktivität zu senken, deren Energieverbrauch zu erhöhen, deren optische Dichte oder Zahl wachstumsfähiger Zellen zu senken, deren biotechnologische Stoffwechselaktivität oder Produktivität zu verringern oder zu inhibieren und/oder direkt zu deren Absterben und Lyse zu führen. In einer bevorzugten Ausführungsform wird wenigstens eine dieser Wirkungen bei einer toxischen Verbindung bereits in geringer Konzentration, vorzugsweise bei einer Konzentration von 1000, bevorzugter 100, noch bevorzugter 50 oder 25, am bevorzugtesten 5 mg/L, erreicht. Dem Fachmann sind zahlreiche routinemäßig anwendbare Verfahren bekannt, mittels derer die Toxizität untersucht werden kann. Hierzu zählen beispielsweise die Messung der Atmung entsprechender Mikroorganismen über $O_2$-Elektroden oder Abgasanalytik oder das vergleichende Ausplattieren von Mikroorganismenproben und das anschließende Zählen der koloniebildenden Einheiten (cfus). In einer bevorzugten Ausführungsform wird unter einer "mäßigen toxischen Wirkung" verstanden, dass in einer Wachstumsphase befindliche Mikroorganismen in Anwesenheit der Verbindung weiter wachsen und/oder stoffwechselaktiv sind, jedoch in geringerem Maße als bei einer Kontrolle, die unter gleichen Bedingungen in Abwesenheit der entsprechenden Verbindung inkubiert wird, und/oder eine verlängerte Lag-Phase aufweisen.

Das Kontaktieren von wässriger und organischer Lösung findet unter geeigneten Bedingungen und insbesondere über einen Zeitraum statt, der für einen ausreichenden Übertritt der organischen Verbindung aus der wässrigen Phase in die organische Phase ausreicht, idealerweise sogar für die Einstellung des entsprechenden Gleichgewichts. Diese Zeitdauer und Bedingungen kann der Fachmann im Rahmen routinemäßigen Experimentierens bestimmen. In einer bevorzugten Ausführungsform dauert Schritt b) wenigstens 0,25, 0,5, 1, 2, 3, 4, 5, 6, 7, 8, 9, 10, 12, 18, 24, 36 oder 48 Stunden oder länger als eine solche Zeitdauer an. Zusätzliche Angaben zur möglichen Wegen zur Ausführung der Erfindung sind in der EP11154707 beschrieben.

Die Temperatur bei Schritt b) hängt nicht nur von den Eigenschaften des flüssigen Kationenaustauschers ab, sondern, insbesondere für den Fall, dass das Kontaktieren der wässrigen und der organischen Lösung bei ablaufender Reaktion in der wässrigen Phase stattfindet, auch von den Temperaturerfordernissen etwaiger, in der wässrigen Phase stattfindenden Reaktionen. Insbesondere für den Fall, dass eine stoffwechselaktive Zelle in der wässrigen Phase katalytisch aktiv ist, muss die Temperatur für die Erhaltung dieser Aktivität geeignet sein. In einer bevorzugten Ausführungsform beträgt die Temperatur bei Schritt b) 0 bis 100 °C, bevorzugter 20 bis 80 °C, 28 bis 70 °C, 30 bis 37 °C, 35 bis 40 °C.

Auch der pH-Wert bei Schritt b) muss den Erfordernissen etwaiger gleichzeitig ablaufender Reaktionen, des Stoffübergangs der gelösten, organischen Verbindung in die Phase des flüssigen lonenaustauschers, der Stabilität von Edukten, Produkten, Zwischenprodukten oder Agenzien Rechnung tragen. In einer bevorzugten Ausführungsform beträgt der pH-Wert 3 bis 8, bevorzugter 6 bis 8, noch bevorzugter 6,2 bis 7,2. Ggf. ist es notwendig, den pH-Wert in der wässrigen Lösung konstant zu halten bzw. anderweitig zu steuern oder zu regeln und damit Korrekturmittel (z.B. Ammoniakwasser/-gas, Schwefelsäure o.ä.) zuzuführen.

Um die organische Verbindung aus der wässrigen Phase möglichst vollständig in die organische zu überführen, ist eine ausreichende Menge des flüssigen hydrophoben Kationenaustauschers erforderlich. In einer bevorzugten Ausführungsform der vorliegenden Erfindung beträgt das Stoffmengenverhältnis von flüssigem Kationenaustauscher und organischer Verbindung bei wenigstens einem Schritt, bei einem kontinuierlichen Prozess über den gesamten Ablauf der Reaktion summiert, wenigstens 1, d. h. pro Molekül der organischen Verbindung wird wenigstens ein Molekül flüssiger hydrophober Kationenaustauscher eingesetzt. In einer noch bevorzugteren Ausführungsform ist das Verhältnis größer als 2, 3, 5, 10, 15, oder 20, bevorzugt 1,5 bis 3.

Das Volumenverhältnis der organischen Lösung zur wässrigen Lösung ist, zusammen mit dem Stoffmengenverhältnis Kationenaustauscher/organische Verbindung, für ein effizientes Verfahren bedeutend. In einer besonderen Ausführungsform beträgt es 100:1 bis 1:100, bevorzugter 20:1 bis 1:20, noch bevorzugter 10:1 bis 1:10, 4:1 bis 1:4, 3:1 bis 1:3 oder am bevorzugtesten 1:2 bis 2:1.

Neben dem flüssigen hydrophoben Kationenaustauscher kann die hydrophobe organische Phase weiterhin ein hydrophobes Lösungsmittel enthalten. Dies kann dazu dienen, die Aufnahmekapazität eines flüssigen hydrophoben Kationenaustauschers in der hydrophoben Phase zu erhöhen und unerwünschtes Verhalten, beispielsweise Ausflocken, zu verhindern. In einer bevorzugten Ausführungsform handelt es sich bei dem Lösungsmittel um ein Edukt einer in der wässrigen Lösung ablaufenden Reaktion, am stärksten bevorzugt das Substrat einer in der wässrigen Lösung ablaufenden enzymkatalysierten Reaktion. In einer bevorzugten Ausführungsform handelt es sich um einen Fettsäureester. In einer besonders bevorzugten Ausführungsform handelt es sich bei dem Lösungsmittel um einen Fettsäureester, bevorzugt - methylester, einer Fettsäure, die als flüssiger hydrophober Kationenaustauscher dient. Es können auch Mischungen von mehr als einem Lösungsmittel und einem oder mehr als einem flüssigen hydrophoben Kationenaustauscher als hydrophobe organische Lösung verwendet werden.

Der Anteil des Lösungsmittels, sofern vorhanden, an der hydrophoben organischen Phase beträgt in einer bevorzugten Ausführungsform 1 bis 99 Volumenprozent (Vol.-%). In einer bevorzugten Ausführungsform beträgt der Anteil des Lösungsmittels 10 bis 90, noch bevorzugter 20 bis 80, am bevorzugtesten 25 bis 75 Vol.-%.

In einer bevorzugten Ausführungsform handelt es sich bei der stoffwechselaktiven Zelle um eine rekombinante Zelle, die mit Enzymen zur Herstellung der Verbindung der Formel (I) oder (II) ausgestattet ist und wenigstens eines davon, bevorzugt alle überexprimieren. Geeignete Enzyme, die zur Herstellung von organischen Verbindungen der Formel (I) oder (II) verwendet werden können, insbesondere Alkanhydroxylasen, AlkL, Transaminasen, Aldehyddehydrogenasen und Alanindehydrogenasen sind im Stand der Technik beschrieben, beispielsweise in der DE102007060705, der EP11004029 oder in der PCT/EP2011/053834. In einer bevorzugten Ausführungsform wird unter dem Begriff "stoffwechselaktive Zelle", wie hierin verwendet, eine lebende Zelle mit Stoffwechselaktivität verstanden, bevorzugt eine Zelle, die ein für die biotechnologische Herstellung des interessierenden Produktes relevantes Enzym in aktiver Form exprimiert oder noch bevorzugter überexprimiert. Bei der Zelle kann es sich um einen Prokaryonten, einschließlich Archaeen, oder um einen Eukaryonten handeln, im Falle eines Prokaryonten bevorzugt aus der Gruppe von Gattungen umfassend *Pseudomonas, Corynebacterium* und *Escherichia.* In einer noch bevorzugteren Ausführungsform handelt es sich bei der Zelle um eine bakterielle Zelle, noch bevorzugter um eine Gram-negative bakterielle Zelle, am bevorzugtesten um *E. coli.* In einer weiteren bevorzugten Ausführungsform handelt es sich um eine eukaryontische Zelle, bevorzugter um eine Pilzzelle, noch bevorzugter um eine Hefezelle, am bevorzugtesten um *Saccharomyces* oder *Candida, Pichia, insbesondere Candida tropicalis.* In einer bevorzugten Ausführungsform bedeutet der Begriff "niederer Eukaryont", wie hierin verwendet, ein in sämtlichen Phasen seiner Existenz unizellulärer Eukaryont, im Gegensatz zu höheren Eukaryonten, die den überwiegenden Teil ihres Lebens in Form eines mehrzelligen Organismus mit Geweben umfassend differenzierte Zellen verbringen. Der Begriff "Zelle" wird, in einer besonderen Ausführungsform, in dieser Anmeldung gleichbedeutend und austauschbar mit dem Begriff "Mikroorganismus" verwendet. Weiterhin kann es sich bei der Zelle um eine isolierte Zelle oder eine Mischung verschiedener Zellen handeln.

Das Verfahren kann verwendet werden, um Fettsäuren oder deren Ester zunächst zu oxidieren und anschließend zu aminieren. Dazu eignet sich beispielsweise ein Enzymsystem, wie es in der internationalen Patentanmeldung WO 2009/077461 beschrieben ist. Bei der stoffwechselaktiven Zelle handelt es sich in diesem Fall um eine Zelle, die eine rekombinante Alkanhydroxylase und eine Transaminase aufweist, bevorzugt darüber hinaus wenigstens ein Enzym aus der Gruppe umfassend Alkoholdehydrogenase, Alanindehydrogenase und Lactamhydrolase.

In der bevorzugtesten Ausführungsform handelt es sich bei der Alkanhydroxylase um eine Alkanhydroxylase des AlkB-Typs. AlkB stellt eine Oxidoreduktase aus dem AlkBGT-System aus *Pseudomonas putida* dar, die für ihre Hydroxylase-Aktivität bekannt ist. Diese ist von zwei weiteren Polypeptiden, AlkG und AlkT abhängig, die bevorzugt co-exprimiert werden. AlkT wird als FAD-abhängige Rubredoxin-Reduktase charakterisiert, die Elektronen aus NADH an AlkG weitergibt. AlkG ist ein Rubredoxin, ein eisenhaltiges Redoxprotein, das als direkter Elektronendonor für AlkB fungiert. In einer bevorzugten Ausführungsform wird unter dem Begriff "Alkanhydroxylase des alkB-Typs", wie hierin

verwendet, eine membranständige Alkanmonooxidase verstanden. In einer weiteren bevorzugten Ausführungsform wird unter dem selben Begriff "Alkanhydroxylase des alkB-Typs" ein Polypeptid mit einer Sequenzhomologie von zunehmend bevorzugt wenigstens 75, 80, 85, 90, 92, 94, 96, 98 oder 99 % zur Sequenz des AlkB von *Pseudomonas putida* Gpo1 (Datenbankcode: CAB54050.1) verstanden. In einer weiteren bevorzugten Ausführungsform wird unter dem Begriff eine cytochromunabhängige Monooxygenase verstanden. In einer weiteren bevorzugten Ausführungsform wird unter dem Begriff "Alkanhydroxylase des alkB-Typs" 5 eine cytochromunabhängige Monooxygenase verstanden, die wenigstens ein Rubredoxin oder Homologon als Elektronendonor verwendet. In einer besonders bevorzugten Ausführungsform wird unter dem Begriff eine membranständige, cytochromunabhänige Alkanhydroxylase mit zunehmend bevorzugt wenigstens 60, 70, 80, 80, 85, 90, 92, 94, 96, 98 oder 99 % zur Sequenz des AlkB von *Pseudomonas putida* Gpo1 verstanden, die als Elektronendonor wenigstens AlkG (CAB54052.1), bevorzugt aber die Kombination von AlkG mit der Reduktase AlkT (CAB54063.1) benötigt, wobei es sich bei alkG und/oder alkT auch um ein Homologon des jeweiligen Polypeptides handeln kann. Der Begriff "Sequenz", wie hierin verwendet, kann sich auf die Aminosäuresequenz eines Polypeptides und/oder die dafür codierende Nukleinsäuresequenz beziehen. In einer weiteren bevorzugten Ausführungsform handelt es sich bei einer "Alkanhydroxylase des alkB-Typs", wie hierin verwendet, um eine Cytochrom-unabhängige Oxidoreduktase, d.h. eine Oxidoreduktase, die nicht Cytochrom als Cofaktor umfasst. Bei sämtlichen in dieser Anmeldung angegebenen Datenbankcodes handelt es sich um Codes der NCBI-Datenbanken in der am 1. August 2012 online verfügbaren Version.

In einer bevorzugten Ausführungsform wird unter dem Begriff "Alkoholdehydrogenase", wie hierin verwendet, ein Enzym verstanden, das einen Aldehyd bzw. ein Keton zu dem entsprechenden primären bzw. sekundären Alkohol reduziert. Beispiele umfassend die Alkoholdehydrogenasen von *Ralstonia eutropha* (ACB78191.1), *Lactobacillus brevis* (YP_795183.1), *Lactobacillus kefiri* (ACF95832.1), aus Pferdeleber, von *Paracoccus pantotrophus* (ACB78182.1) und *Sphingobium yanoikuyae* (EU427523.1) sowie die jeweiligen Varianten davon.

In einer bevorzugten Ausführungsform wird unter dem Begriff "Transaminase", wie hierin verwendet, ein Enzym verstanden, das die Übertragung von Aminogruppen von einem Donor-, bevorzugt einer Aminosäure, auf ein Akzeptormolekül, bevorzugt eine $\alpha$-Ketocarbonsäure, katalysiert. Beispielsweise kann die Transaminase von *Chromobacterium violaceum* ATCC 12472 (Datenbankcode NP_901695) verwendet werden.

In einer bevorzugten Ausführungsform wird unter dem Begriff "Alanindehydrogenase", wie hierein verwendet, ein Enzym verstanden, das die Umwandlung von L-Alanin unter Verbrauch von Wasser und $NAD^+$ zu Pyruvat, Ammoniak und NADH katalysiert. Beispielsweise können die Alanindehydrogenasen aus *Bacillus subtilis* (Datenbankcode L20916), *Rhizobium leguminosarum* (Datenbankcode CP001622), *Vibrio proteolytikus* (Datenbankcode AF070716), *Mycobacterium tuberculosis* (Datenbankcode X63069), *Enterobacter aerogenes* (Datenbankcode AB013821) verwendet werden.

In der bevorzugtesten Ausführungsform handelt es sich bei der Alkanhydroxylase um eine Alkanhydroxylase des AlkB-Typs. AlkB stellt eine Oxidoreduktase aus dem AlkBGT-System aus *Pseudomonas putida* dar, die für ihre Hydroxylase-Aktivität bekannt ist. Diese ist von zwei weiteren Polypeptiden, AlkG und AlkT abhängig, die bevorzugt co-exprimiert werden. AlkT wird als FAD-abhängige Rubredoxin-Reduktase charakterisiert, die Elektronen aus NADH an AlkG weitergibt. AlkG ist ein Rubredoxin, ein eisenhaltiges Redoxprotein, das als direkter Elektronendonor für AlkB fungiert. In einer bevorzugten Ausführungsform wird unter dem Begriff "Alkanhydroxylase des alkB-Typs", wie hierin verwendet, eine membranständige Alkanmonooxidase verstanden. In einer weiteren bevorzugten Ausführungsform wird unter dem selben Begriff "Alkanhydroxylase des alkB-Typs" ein Polypeptid mit einer Sequenzhomologie von zunehmend bevorzugt wenigstens 75, 80, 85, 90, 92, 94, 96, 98 oder 99 % zur Sequenz des AlkB von *Pseudomonas putida* Gpo1 (Datenbankcode: CAB54050.1) verstanden. In einer weiteren bevorzugten Ausführungsform wird unter dem Begriff eine Cytochrom-unabhängige Monooxygenase verstanden. In einer weiteren bevorzugten Ausführungsform wird unter dem Begriff "Alkanhydroxylase des alkB-Typs" 5 eine cytochromunabhängige Monooxygenase verstanden, die wenigstens ein Rubredoxin oder Homologon als Elektronendonor verwendet. In einer besonders bevorzugten Ausführungsform wird unter dem Begriff eine membranständige, cytochromunabhänige Alkanhydroxylase mit zunehmend bevorzugt wenigstens 60, 70, 80, 80, 85, 90, 92, 94, 96, 98 oder 99 % zur Sequenz des AlkB von *Pseudomonas putida* Gpo1 verstanden, die als Elektronendonor wenigstens AlkG (CAB54052.1), bevorzugt aber die Kombination von AlkG mit der Reduktase AlkT (CAB54063.1) benötigt, wobei es sich bei alkG und/oder alkT auch um ein Homologon des jeweiligen Polypeptides handeln kann. Der Begriff "Sequenz", wie hierin verwendet, kann sich auf die Aminosäuresequenz eines Polypeptides und/oder die dafür codierende Nukleinsäuresequenz beziehen. In einer weiteren bevorzugten Ausführungsform handelt es sich bei einer "Alkanhydroxylase des alkB-Typs", wie hierin verwendet, um eine Cytochrom-unabhängige Oxidoreduktase, d.h. eine Oxidoreduktase, die nicht Cytochrom als Cofaktor umfasst.

In einer weiteren bevorzugten Ausführungsform handelt es sich bei der Alkanhydroxylase um eine Cytochrom P450-Monooxygenase der CYP153-Familie. In einer bevorzugten Ausführungsform wird unter dem Begriff "Cytochrom P450-Monooxygenase der CYP153-Familie" eine cytosolische Oxidase verstanden, die Teil eines 3 Komponenten-Systems ist, das weiterhin ein Ferredoxin und eine Ferredoxin-Reduktase umfasst, mit einer Alkan-Bindestelle und der Fähigkeit, Alkane zu hydroxylieren. In einer besonders bevorzugten Ausführungsform handelt es sich um ein Enzym, das zu wenigstens 80, bevorzugt 90, am bevorzugtesten 95 oder 99 Prozent Sequenzidentität zur Cytochrom P450-Monooxygenase der CYP153-Familie aus *Alcanivorax borkumensis* SK2 (Datenbankcode YP_691921) aufweist oder um ein Enzym, das eine Polypeptidsequenz umfasst, die wenigstens 80, bevorzugt 90, am bevorzugtesten 95 oder 99 Prozent Sequenzidentität zur Cytochrom P450-Monooxygenase der CYP153-Familie aus *Alcanivorax borkumensis* SK2 (Datenbankcode YP_691921) aufweist und darüber hinaus Alkanhydroxylase-Aktivität aufweist. In einer bevorzugten Ausführungsform ist unter dem Begriff "Alkanhydroxylase-Aktivität", wie hierin verwendet, die Fähigkeit zu verstehen, die Hydroxylierung von Alkanen oder unsubstituierten linearen Alkylresten umfassend wenigstens fünf, bevorzugt zwölf Kohlenstoffstoffreste zu katalysieren. In einer weiteren bevorzugten Ausführungsform wird unter dem Begriff "Cytochrom P450-Monooxygenase der CYP153-Familie" eine nicht membrangebundene Oxidase verstanden, die eine Bindestelle für Alkane, unsubstituierte lineare Alkylreste umfassend wenigstens fünf, bevorzugt zwölf Kohlenstoffreste oder einfach hydroxylierte Alkane und deren Polypeptidkette das Motiv LL(I/L)(V/I)GGNDTTRN umfasst. In einer bevorzugten Ausführungsform handelt es sich bei einer "Cytochrom P450-Monooxygenase der CYP153-Familie", wie hierin verwendet, um eine Cytochrom P450-Monooxygenase der CYP153-Familie aus *Alcanivorax borkumensis* SK2 (Datenbankcode YP_691921) oder eine Variante, die bevorzugt Alkanhydroxylaseaktivität aufweist.

Zur optimalen Versorgung der Cytochrom P450-Monooxygenase der CYP153-Familie mit Elektronen aus dem Reduktionsmittel, bevorzugt NADH, ist es bevorzugt, dass die Monoxygenase zusammen mit funktionell mit ihr wechselwirkender Ferredoxin-Reduktase und funktionell mit ihr wechselwirkendem Ferredoxin verwendet wird. Dabei kann es sich um isolierte oder bei der Verwendung einer stoffwechselaktiven Zelle um co-exprimierte Polypeptide oder um N- oder C-terminal mit der Cytochrom P450-Monooxygenase der CYP153-Familie fusionierte Polypeptide handeln. Ob eine Ferredoxin-Reduktase oder ein Ferredoxin mit einer gegebenen Cytochrom P450-Monooxygenase der CYP153-Familie mit einander funktionell wechselwirken, kann der Fachmann leicht dadurch feststellen, dass das Reduktionsmittel in Gegenwart eines Alkansubstrates und der drei Polypeptide oxidiert wird. Alternativ kann der von Scheps, D., Malca, H., Hoffmann, B., Nestl, B. M, und Hauer, B. (2011) Org. Biomol. Chem., 9, 6727 beschriebene Enzymtest verwendet werden, der im Fall funktionell wechselwirkender Polypeptide eine deutliche Erhöhung der Reaktionsgeschwindigkeit zeigt. In einer besonders bevorzugten Ausführungsform stammen die Cytochrom P450-Monooxygenase der CYP153-Familie, das Ferredoxin und die Ferredoxin-Reduktase aus dem gleichen Organismus. In einer besonders bevorzugten Ausführungsform handelt es sich um die Ferredoxin-Reduktase aus *Alcanivorax borkumensis* SK2 (Datenbankcode YP_691923) oder eine Variante davon, das Ferredoxin aus *Alcanivorax borkumensis* SK2 (Datenbankcode YP_691920) oder eine Variante davon und die Cytochrom P450-Monooxygenase der CYP153-Familie aus *Alcanivorax borkumensis* SK2 (Datenbankcode YP_691921) oder eine Variante davon.

Die Lehre der vorliegenden Erfindung kann nicht nur unter Verwendung der bzw. auf die exakten Aminosäure- oder Nukleinsäuresequenzen der hierin beschriebenen biologischen Makromoleküle ausgeführt bzw. angewendet werden, beispielsweise durch Knock out eines Gens, das für ein eine der Reaktionen der β-Oxidation katalysierendes Enzym kodiert, sondern auch unter Verwendung von bzw. auf Varianten derartiger Makromoleküle, die durch Deletion, Addition oder Substitution einer oder mehr als einer Aminosäuren oder Nukleinsäuren erhalten werden können. In einer bevorzugten Ausführungsform bedeutet der Begriff "Variante" einer Nukleinsäuresequenz oder Aminosäuresequenz, im Folgenden gleichbedeutend und austauschbar mit dem Begriff "Homologon" gebraucht, wie hierin verwendet, eine andere Nukleinsäure- oder Aminosäuresequenz, die mit Hinblick auf die entsprechende ursprüngliche Wildtyp-Nukleinsäure- oder -aminosäuresequenz eine Homologie, hier gleichbedeutend mit Identität verwendet, von 70, 75, 80, 85, 90, 92, 94, 96, 98, 99 % oder mehr Prozent aufweist, wobei bevorzugt andere als die das katalytisch aktive Zentrum ausbildende Aminosäuren oder für die Struktur oder Faltung essentielle Aminosäuren deletiert oder substituiert sind oder letztere lediglich konservativ substituiert sind, beispielsweise ein Glutamat statt einem Aspartat oder ein Leucin statt einem Valin. Der Stand der Technik beschreibt Algorithmen, die verwendet werden können, um das Ausmaß von Homologie von zwei Sequenzen zu berechnen, z. B. Arthur Lesk (2008), Introduction to bioinformatics, 3rd edition. In einer weiteren bevorzugteren Ausführungsform der vorliegenden Erfindung weist die Variante einer Aminosäure- oder Nukleinsäuresequenz, bevorzugt zusätzlich zur oben genannten Sequenzhomologie, im Wesentlichen die gleiche enzymatisch Aktivität des Wildtypmoleküls bzw. des ursprünglichen Moleküls auf. Zum Beispiel weist eine Variante eines als Protease enzymatisch aktiven Polypeptids die gleiche oder im Wesentlichen die gleiche proteolytische Aktivität wie das Polypeptidenzym auf, d.h. die Fähigkeit, die Hydrolyse einer Peptidbindung zu katalysieren. In einer besonderen Ausführungsform bedeutet der Begriff "im Wesentlichen die gleiche enzymatische Aktivität" eine Aktivität mit Hinblick auf die Substrate des Wildtyp-Polypeptids, die deutlich über der Hintergrundaktivität liegt oder/und sich um weniger als 3, bevorzugter 2, noch bevorzugter eine Größenordnung von den $K_M$- und/oder $k_{cat}$- Werten unterscheidet, die das Wildtyppolypeptid mit Hinblick auf die gleichen Substrate aufweist. In einer weiteren bevorzugten Ausführungsform umfasst der Begriff "Variante" einer Nukleinsäure- oder Aminosäuresequenz wenigstens einen aktiven Teil/oder Fragment der Nukleinsäure- bzw. Aminosäuresequenz. In einer weiteren bevorzugten Ausführungsform bedeutet der Begriff "aktiver Teil", wie hierin verwendet, eine Aminosäuresequenz oder eine Nukleinsäuresequenz, die eine geringere als die volle Länge der Aminosäuresequenz aufweist bzw. für eine geringere als die volle Länge der Aminosäuresequenz kodiert, wobei die Aminosäuresequenz oder die kodierte Aminosäuresequenz mit geringerer Länge als der Wildtyp-Aminosäuresequenz im Wesentlichen die gleiche enzymatische Aktivität wie das Wildtyppolypeptid oder eine Variante davon aufweist, beispielsweise als Fettsäureimporter, als Enoyl-CoA-Hydratase bzw. FadE oder als Acetyl-CoA-Acyltransferase bzw. FadB. In einer besonderen Ausführungsform umfasst der Begriff "Variante" einer Nukleinsäure eine Nukleinsäure, deren komplementärer Strang, bevorzugt unter stringenten Bedingungen, an die Wildtyp-Nukleinsäure bindet. Die Stringenz der Hybridisierungsreaktion ist für den Fachmann leicht bestimmbar und hängt im Allgemeinen von der Länge der Sonde, den Temperaturen beim Waschen und der Salzkonzentration ab. Im Allgemeinen benötigen längere Sonden höhere Temperaturen zum Hybridisieren, wohingegen kürzere Proben mit geringen Temperaturen auskommen. Ob Hybridisierung stattfindet, hängt im Allgemeinen von der Fähigkeit der denaturierten DNA ab, an komplementäre Stränge zu annellieren, die in ihrer Umgebung vorhanden sind, und zwar unterhalb der Schmelztemperatur. Die Stringenz von Hybridisierungsreaktion und entsprechende Bedingungen sind ausführlicher in Ausubel *et al.* 1995 beschrieben. Anleitungen zur Identifizierung von DNA-Sequenzen mittels Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology 41: 255-260 (19991)). Die Hybridisierung findet in einer bevorzugten Ausführungsform unter stringenten Bedingungen statt, das heißt, es werden nur Hybride gebildet, bei denen Sonde und Zielsequenz, d. h. die mit der Sonde behandelten Polynukleotide, mindestens 70% identisch sind. Es ist bekannt, dass die Stringenz der Hybridisierung einschließlich der Waschschritte durch Variieren der Pufferzusammensetzung, der Temperatur und der Salzkonzentration beeinflusst bzw. bestimmt wird. Die Hybridisierungsreaktion wird im Allgemeinen bei relativ niedriger Stringenz im Vergleich zu den Waschschritten durchgeführt (Hybaid Hybridisation Guide, Hybaid Limited, Teddington, UK, 1996). Für die Hybridisierungsreaktion kann beispielsweise ein Puffer entsprechend 5x SSC-Puffer bei einer Temperatur von ca. 50°C - 68°C eingesetzt werden. Dabei können Sonden auch mit Polynukleotiden hybridisieren, die weniger als 70% Identität zur Sequenz der Sonde aufweisen. Solche Hybride sind weniger stabil und werden durch Waschen unter stringenten Bedingungen entfernt. Dies kann beispielsweise durch Senken der Salzkonzentration auf 2x SSC und gegebenenfalls nachfolgend 0,5x SSC (The DIG System User's Guide for Filter Hybridisation, Boehringer Mannheim, Mannheim, Deutschland, 1995) erreicht werden, wobei eine Temperatur von in der Reihenfolge zunehmender Bevorzugung ca. 50°C - 68°C, ca. 52°C - 68°C, ca. 54°C - 68°C, ca. 56°C - 68°C, ca. 58°C - 68°C, ca. 60°C - 68°C, ca. 62°C - 68°C, ca. 64°C - 68°C, ca. 66°C - 68°C eingestellt wird. Temperaturbereiche von ca. 64°C - 68°C oder ca. 66°C - 68°C werden bevorzugt. Es ist gegebenenfalls möglich die Salzkonzentration bis auf eine Konzentration entsprechend 0,2 x SSC oder 0,1 x SSC zu senken. Durch schrittweise Erhöhung der Hybridisierungstemperatur in Schritten von ca. 1 - 2°C von 50°C auf 68°C können Polynukleotidfragmente isoliert werden, die beispielsweise in der Reihenfolge zunehmender Bevorzugung mindestens 70% oder mindestens 80% oder mindestens 90%, mindestens 91%, mindestens 92%, mindestens 93%, mindestens 94%, mindestens 95%, mindestens 96%, mindestens 97%, mindestens 98% oder mindestens 99% Identität zur Sequenz des eingesetzten Nukleinsäuremoleküls. Weitere Anleitungen zur Hybridisierung sind in Form sogenannter Kits am Markt erhältlich (z.B. DIG Easy Hyb von der Firma Roche Diagnostics GmbH, Mannheim, Deutschland, Catalog No. 1603558). In einer bevorzugten Ausführungsform umfasst der Begriff "Variante" einer Nukleinsäure, wie hierin verwendet, eine beliebige Nukleinsäuresequenz, die für die gleiche Aminosäuresequenz wie die ursprüngliche Nukleinsäure oder eine Variante dieser Aminosäuresequenz im Rahmen der Degeneriertheit des genetischen Codes kodiert.

Nach dem zweiten Schritt wird die hydrophobe organische Lösung von dem wässrigen Kulturmedium abgetrennt. Dies ist aufgrund der inhärenten Fähigkeit dieses Systems zur Ausbildung zweier Phasen ein für den Fachmann leicht durchzuführender Vorgang, der einfach durch Stehenlassen des Gefäßes und anschließendes Abdekantieren einer Phase vonstatten gehen kann. Alternativ kann ein Scheidetrichter Verwendung finden. Im Falle ausreichend verschiedener Siedepunkte besteht die Möglichkeit, die bei niedrigeren Temperaturen siedende Phase, bei der es sich in der Regel um die organische Phase handeln wird, durch das Anlegen von Unterdruck abzuziehen. Geringe Mengen von in der organischen Phase verbliebenem Wasser können unter Verwendung von anorganischen Trockenmitteln wie Calciumhydrid, wasserfreiem Calciumchlorid, Kieselgel, wasserfreiem Natriumsulfat, Natriumhydroxid oder dergleichen entfernt werden.

Weitere Anleitungen zur Ausführung der Erfindung können der PCT/EP2011/071491 entnommen werden, wobei die darin beschriebenen Fettsäuren durch die erfindungsgemäßen zu ersetzen sind.

**Patentansprüche**

1. Verfahren zum Entfernen einer organischen Verbindung aus einer wässrigen Lösung, umfassend die Schritte

   a) Bereitstellen der wässrigen Lösung, die die organische Verbindung enthält, und einer hydrophoben organischen Lösung, wobei letztere einen flüssigen hydrophoben Kationenaustauscher umfasst,
   b) Kontaktieren der wässrigen Lösung und der hydrophoben organischen Lösung, und
   c) Abtrennen der hydrophoben organischen Lösung von der wässrigen Lösung,
   wobei es sich bei dem flüssigen hydrophoben Kationenaustauscher um eine gesättigte Alkansäure mit wenigstens einem Alkylsubstituenten handelt,
wobei es sich bei der organischen Verbindung um eine organische Verbindung mit wenigstens einer positiven Ladung und einer neutralen oder positiven Gesamtladung handelt.

2. Verfahren nach Anspruch 1, wobei es sich bei der organischen Verbindung um eine Verbindung der Formel

$$NR^2R^3R^4H^+\text{-}A\text{-}COOR^1 \qquad (I)$$

oder

$$NR^2R^3R^4H^+\text{-}A\text{-} NR^5R^6R^7H^+ \qquad (II),$$

handelt,
wobei $R^1$ Wasserstoff, Methyl, Ethyl, Propyl, Isopropyl oder eine negative Ladung ist,
wobei A eine Alkylengruppe mit wenigstens drei, bevorzugt wenigstens sechs, besonders bevorzugt acht Kohlenstoffatomen ist, die bevorzugt unsubstituiert und geradkettig ist
und wobei $R^2$, $R^3$, $R^4$, $R^5$, $R^6$ und $R^7$ jeweils und unabhängig voneinander aus der Gruppe ausgewählt sind, die Wasserstoff, Methyl, Ethyl und Propyl umfasst.

3. Verfahren nach einem der Ansprüche 1 bis 2, wobei der pH-Wert in der wässrigen Lösung bei Schritt b) 6 bis 8, bevorzugt 6,2 bis 7,2, beträgt.

4. Verfahren nach einem der Ansprüche 1 bis 3, wobei das Stoffmengenverhältnis von flüssigem Kationenaustauscher zu organischer Verbindung bei Schritt b) wenigstens 1 beträgt.

5. Verfahren nach einem der Ansprüche 1 bis 4, wobei das Volumenverhältnis von organischer Lösung zu wässriger Lösung 1:10 bis 10:1 beträgt.

6. Verfahren nach einem der Ansprüche 1 bis 5, wobei der flüssige Kationenaustauscher eine verzweigte Fettsäure der Formel $(H_3C)_2CH\text{-}(CH_2)_n\text{-}COOH$ oder eine unprotonierte Form davon ist und n wenigstens 4, bevorzugt wenigstens 8 beträgt und am bevorzugtesten 14 ist.

7. Verfahren nach einem der Ansprüche 1 bis 6, wobei der flüssige Kationenaustauscher eine gesättigte Alkansäure mit wenigstens einem Alkylsubstituent ist, die bevorzugt insgesamt wenigstens 12 Kohlenstoffatome umfasst.

8. Verfahren nach einem der Ansprüche 1 bis 7, wobei die wässrige Lösung weiterhin eine stoffwechselaktive Zelle umfasst.

9. Verfahren nach Anspruch 8, es sich bei der organischen Verbindung um eine für eine Zelle, bevorzugt eine bakterielle Zelle, toxische Verbindung handelt.

10. Verfahren nach einem der Ansprüche 1 bis 9, wobei die organische Lösung darüber hinaus mindestens ein organisches Lösungsmittel enthält, bevorzugt eine Fettsäure und/oder einen Fettsäureester.

11. Verfahren nach Anspruch 10, wobei die hydrophobe organische Lösung den flüssigen hydrophoben Kationenaustauscher in einem Volumenanteil von 20 bis 80 %, bevorzugt 25 bis 75 % enthält.

12. Verfahren nach einem der Ansprüche 1 bis 11, wobei es sich bei der organischen Verbindung um ein Diamin aus der Gruppe handelt, die 1,4-Butandiamin, 1,5-Pentandiamin, 1,6-Hexandiamin, 1,8-Octandiamin, 1,14-Tetradecandiamin, 1,18-Octadecandiamin, 2-Methyl-1,5-Diaminopentan, 2,2-Dimethyl-1,5-diaminopentan, 4,4'-Diaminodicyclohexylmethan, 3,3'-Dimethyl-4,4'-diaminodicyclohexylmethan, 3,3',5,5'-Tetramethyl-4,4'-diaminodicyclohexylmethan, m- oder p-Xylylendiamin, 2.2.4- oder 2.4.4-Trimethylhexamethylendiamin, 1.4-Diaminocyclohexan, 4.4'-Diaminodicyclohexylpropan, Isophorondiamin, Metaxylylendiamin und Paraxylylendiamin umfasst.

13. Verfahren nach einem der Ansprüche 1 bis 12, wobei die Temperatur bei Schritt b) zwischen 28 und 70 °C, bevorzugt zwischen 30 und 37 °C liegt.

14. Reaktionsmischung umfassend eine wässrige Lösung, die eine organische Verbindung enthält, und eine hydrophobe organische Lösung,

wobei die hydrophobe organische Lösung einen flüssigen hydrophoben Kationenaustauscher umfasst,

wobei es sich bei dem flüssigen hydrophoben Kationenaustauscher um eine gesättigte Alkansäure mit wenigstens einem Alkylsubstituenten handelt,

und wobei es sich bei der organischen Verbindung um eine organische Verbindung mit wenigstens einer positiven Ladung und einer neutralen oder positiven Gesamtladung handelt.

15. Reaktionsmischung nach Anspruch 14, wobei es sich bei der organischen Verbindung um eine Verbindung der Formel

$$NR^2R^3R^4H^+\text{-A-COOR}^1 \qquad (I)$$

oder

$$NR^2R^3R^4H^+\text{-A- }NR^5R^6R^7H^+ \qquad (II),$$

handelt,

wobei $R^1$ Wasserstoff, Methyl, Ethyl, Propyl, Isopropyl oder eine negative Ladung ist,

wobei A eine Alkylengruppe mit wenigstens drei, bevorzugt wenigstens sechs, besonders bevorzugt acht Kohlenstoffatomen ist, die bevorzugt unsubstituiert und geradkettig ist

und wobei $R^2$, $R^3$, $R^4$, $R^5$, $R^6$ und $R^7$ jeweils und unabhängig voneinander aus der Gruppe ausgewählt sind, die Wasserstoff, Methyl, Ethyl und Propyl umfasst.

16. Reaktionsmischung nach einem der Ansprüche 14 bis 15, wobei der flüssige hydrophobe Kationenaustauscher eine verzweigte Fettsäure der Formel $(H_3C)_2CH\text{-}(CH_2)_n\text{-COOH}$ oder eine unprotonierte Form davon ist und n wenigstens 4, bevorzugt wenigstens 8 beträgt und am bevorzugtesten 14 ist.

17. Reaktionsmischung nach einem der Ansprüche 14 bis 16, wobei der flüssige Kationenaustauscher eine gesättigte Alkansäure mit wenigstens einem Alkylsubstituenten ist, die bevorzugt insgesamt wenigstens 12 Kohlenstoffatome umfasst.

18. Reaktionsmischung nach einem der Ansprüche 14 bis 17, wobei die wässrige Lösung weiterhin eine stoffwechselaktive Zelle umfasst.

19. Reaktionsmischung nach einem der Ansprüche 14 bis 18, wobei es sich bei der organischen Verbindung um ein Diamin aus der Gruppe handelt, die Butandiamin, 1,5-Pentandiamin, 1,6-Hexandiamin, 1,8-Octandiamin, 1,14-Tetradecandiamin, 1,18-Octadecandiamin, 2-Methyl-1,5-Diaminopentan, 2,2-Dimethyl-1,5-diaminopentan, 4,4'-Diaminodicyclohexylmethan, 3,3'-Dimethyl-4,4'-diaminodicyclohexylmethan, 3,3',5,5'-Tetramethyl-4,4'-diaminodicyclohexylmethan, m- oder p-Xylylendiamin, 2.2.4- oder 2.4.4-Trimethylhexamethylendiamin, 1.4-Diaminocyclohexan, 4.4'-Diaminodicyclohexylpropan, Isophorondiamin, Metaxylylendiamin und Paraxylylendiamin umfasst.

Europäisches
Patentamt

European
Patent Office

Office européen
des brevets

## EUROPÄISCHER RECHERCHENBERICHT

Nummer der Anmeldung

EP 12 18 1153

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 0 049 429 A1 (MILES LAB [US]) 14. April 1982 (1982-04-14)  * Seite 14, Zeile 20 - Seite 15, Zeile 10; Beispiel 9 * * Seite 2, Zeile 19 - Zeile 24 * ----- | 1,3,5, 7-10,13, 14,17,18 | INV. B01J39/04 B01J39/16 B01D11/04 C02F1/42 C07C227/40 |
| A | PATRICIA D. MACKENZIE ET AL: "Combined solvent extraction and stripping for removal and isolation of ammonia from sour waters", INDUSTRIAL & ENGINEERING CHEMISTRY PROCESS DESIGN AND DEVELOPMENT, Bd. 24, Nr. 4, 1. Oktober 1985 (1985-10-01), Seiten 1192-1200, XP055054507, ISSN: 0196-4305, DOI: 10.1021/i200031a050 * das ganze Dokument * ----- | 1-19 | |
| A | WO 98/02411 A1 (YISSUM RES DEV CO [IL]; WHALLEY KEVIN [GB]; EYAL AHARON MEIR [IL]; COH) 22. Januar 1998 (1998-01-22) * das ganze Dokument * ----- | 1-19 | RECHERCHIERTE SACHGEBIETE (IPC)  B01J B01D C02F C07C |

Der vorliegende Recherchenbericht wurde für alle Patentansprüche erstellt

| Recherchenort | Abschlußdatum der Recherche | Prüfer |
|---|---|---|
| Den Haag | 26. Februar 2013 | Klemps, Christian |

KATEGORIE DER GENANNTEN DOKUMENTE

X : von besonderer Bedeutung allein betrachtet
Y : von besonderer Bedeutung in Verbindung mit einer
　　anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
　　nach dem Anmeldedatum veröffentlicht worden ist
D : in der Anmeldung angeführtes Dokument
L : aus anderen Gründen angeführtes Dokument
...........................................................................
& : Mitglied der gleichen Patentfamilie, übereinstimmendes
　　Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 12 18 1153

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

26-02-2013

| Im Recherchenbericht angeführtes Patentdokument | | Datum der Veröffentlichung | Mitglied(er) der Patentfamilie | | Datum der Veröffentlichung |
|---|---|---|---|---|---|
| EP 0049429 | A1 | 14-04-1982 | AU | 527657 B2 | 17-03-1983 |
| | | | AU | 7603181 A | 29-07-1982 |
| | | | CA | 1183159 A1 | 26-02-1985 |
| | | | DE | 3166050 D1 | 18-10-1984 |
| | | | EP | 0049429 A1 | 14-04-1982 |
| | | | IE | 52126 B1 | 24-06-1987 |
| | | | JP | 57072935 A | 07-05-1982 |
| | | | JP | 59040375 B | 29-09-1984 |
| | | | MX | 152169 A | 04-06-1985 |
| | | | US | 4334095 A | 08-06-1982 |
| | | | ZA | 8103904 A | 25-08-1982 |
| WO 9802411 | A1 | 22-01-1998 | AU | 3455397 A | 09-02-1998 |
| | | | IL | 118855 A | 22-09-1999 |
| | | | US | 6171501 B1 | 09-01-2001 |
| | | | WO | 9802411 A1 | 22-01-1998 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10200710060705
- EP 11154707 A
- EP 11004029 A
- EP 2011053834 W
- WO 2009077461 A
- EP 2011071491 W

**In der Beschreibung aufgeführte Nicht-Patentliteratur**

- **ASANO, Y. ; FUKUTA, Y. ; YOSHIDA, Y. ; KOMEDA, H.** The Screening, Characterisation, and Use of ω-Laurolactam Hydrolase: A New Enzymatic Synthesis of 12-Aminolauric Acid. *Biosc. Biotechn. Biochem.,* 2008, vol. 72 (8), 2141-2150
- **FUCHS ; SCHLEGEL.** Allgemeine Mikrobiologie. Georg Thieme Verlag, 2007
- **J. SANGSTER.** Octanol-Water Partition Coefficients: Fundamentals and Physical Chemistry. Wiley Series in Solution Chemistry, John Wiley & Sons, 1997, vol. 2
- **SCHEPS, D. ; MALCA, H. ; HOFFMANN, B. ; NESTL, B. M ; HAUER, B.** *Org. Biomol. Chem.,* 2011, vol. 9, 6727
- **ARTHUR LESK.** Introduction to bioinformatics. 2008
- The DIG System Users Guide for Filter Hybridization. Firma Boehringer Mannheim GmbH, 1993
- **LIEBL et al.** *International Journal of Systematic Bacteriology,* 1991, vol. 41, 255-260
- Hybaid Hybridisation Guide. Hybaid Limited, 1996
- The DIG System User's Guide for Filter Hybridisation. Boehringer Mannheim, 1995